# EUROPÄISCHE PATENTANMELDUNG

(11) **EP 0 826 772 A2**
(43) Veröffentlichungstag der Anmeldung: **04.03.1998**
(21) Anmeldenummer: 96118422.3
(22) Anmeldetag: 16.11.1996
(51) Int. Cl.: C12N 15/12, C07K 14/47, G01N 33/577, C12Q 1/68, C07K 16/18

(54) **Humanes Muzin (MUC8)**

(30) Priorität: 19.12.1995 DE 19547324
(71) Anmelder: Behring Diagnostics GmbH, 35001 Marburg (DE)
(72) Erfinder: Dippold, Wolfgang, Prof. Dr., 55131 Mainz (DE); Walter, Anette, Menlo Park CA 94025 (US)

(57) **Zusammenfassung**

Die vorliegende Erfindung betrifft die Isolierung und Charakterisierung von Aminosäuren- und Nukleotidsequenzen eines neuen Mitglieds der Muzingenfamilie. Insbesondere betrifft die Erfindung die Bereitstellung von Reagenzien für die Diagnostik von Patienten und für die Vakzinierung zur Behandlung bestimmter Erkrankungen durch Stimulierung der Immunabwehr.

(1) ALLGEMEINE ANGABEN:

(i) ANMELDER:
(A) NAME: Behringwerke Aktiengesellschaft
(B) STRASSE: Emil-von-Behring-Str. 78
(C) ORT: Marburg
(E) LAND: Deutschland
(F) POSTLEITZAHL: 35001
(G) TELEFON: 06421-39-2332
(H) TELEFAX: 06421-39-4558

(ii) BEZEICHNUNG DER ERFINDUNG: Humanes Muzin (MUC8)

(iii) ANZAHL DER SEQUENZEN: 5

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(vi) DATEN DER URANMELDUNG:
(A) ANMELDENUMMER: DE 19547324.8
(B) ANMELDETAG: 19-DEC-1995

(2) ANGABEN ZU SEQ ID NO: 1:

(i) SEQUENZKENNZEICHEN:
(A) LÄNGE: 798 Basenpaare
(B) ART: Nucleotid
(C) STRANGFORM: Doppelstrang
(D) TOPOLOGIE: linear

(ii) ART DES MOLEKÜLS: cDNA

(iii) HYPOTHETISCH: NEIN

(iv) ANTISENSE: NEIN

(vi) URSPRÜNLICHE HERKUNFT:
(A) ORGANISMUS: not known
(F) GEWEBETYP: human colon carcinoma cell line T 84

(vii) UNMITTELBARE HERKUNFT:
(A) BIBLIOTHEK: cDNA-Genbank d. Zellinie
T84(Fa.Stratagene,HD),expr. library
(B) CLON(E): pB1 cDNA (Vektor: Uni-ZAP XR)

(viii) POSITION IM GENOM:
(A) CHROMOSOM/SEGMENT: single copy chromosome 7

(ix) MERKMAL:
(A) NAME/SCHLÜSSEL: CDS
(B) LAGE:1..513

(xi) SEQUENZBESCHREIBUNG: SEQ ID NO: 1:

GCA CGA GAG AAG AGG AAG CCG CAG CAG CCC CAG CGC CGA CCA GCA GGG 48
Ala Arg Glu Lys Arg Lys Pro Gln Gln Pro Gln Arg Arg Pro Ala Gly

1                    5                   10                   15

GGC ACA GGA CAG AGA AGA GGA AGC GGC TAC AGC CCC AGC GCC GAC CAG          96
Gly Thr Gly Gln Arg Arg Gly Ser Gly Tyr Ser Pro Ser Ala Asp Gln
            20                  25                  30

CAG GGG GCA CAG GAC AGA GAA GAG GAA GCC GCA GCA GCC CCA GCG CCG         144
Gln Gly Ala Gln Asp Arg Glu Glu Glu Ala Ala Ala Ala Pro Ala Pro
        35                  40                  45

ACC AGC AGC GGG CAC AGG ACA GAG AAG AGG AAG CGG CTG CAG CTC CAG         192
Thr Ser Ser Gly His Arg Thr Glu Lys Arg Lys Arg Leu Gln Leu Gln
    50                  55                  60

TGC CAA CCA GCA GGG GGC ACA GGA CAG AGA AGA GGA AGC GGC CAA GGC         240
Cys Gln Pro Ala Gly Gly Thr Gly Gln Arg Arg Gly Ser Gly Gln Gly
65                  70                  75                  80

CCC AGT GCC CGA CCA GCA GCT TTC ACA GGA CAG AGA AGA GGA AGC CGC         288
Pro Ser Ala Arg Pro Ala Ala Phe Thr Gly Gln Arg Arg Gly Ser Arg
                85                  90                  95

AGC AGC CCC AGC GCC GAC CAG CAG CGG GCA CAG GAC AGA GAA GAG GAA         336
Ser Ser Pro Ser Ala Asp Gln Gln Arg Ala Gln Asp Arg Glu Glu Glu
            100                 105                 110

GCG GCC AGG CCC CAG CGC CGA CCA GCA GCG GGC ACA GGA CAG AGA AGA         384
Ala Ala Arg Pro Gln Arg Arg Pro Ala Ala Gly Thr Gly Gln Arg Arg
        115                 120                 125

GGA AGC GCA GCA GCC CCA GTG CCG ACC AGC AGC GGC ACA GGA CAG AGA         432
Gly Ser Ala Ala Ala Pro Val Pro Thr Ser Ser Gly Thr Gly Gln Arg
    130                 135                 140

AGA GGA AGC GCA GCA GCC CCA GCG CCG ACC AGC AGC GGC ACA GGA CAG         480
Arg Gly Ser Ala Ala Ala Pro Ala Pro Thr Ser Ser Gly Thr Gly Gln
145                 150                 155                 160

AGA AGA GGA AGT GAG GAA ATG GAA GAA GAG GGT TGACCTGAGC TGAAGGACAT       533
Arg Arg Gly Ser Glu Glu Met Glu Glu Glu Gly
                165                 170

GAACTTCCAC GTGAAATAGC CCCAGGGCCG GGCACACGAG GTCAGGAGTT CAAGACCAGC       593

CTGGCCAAGA TGGTGAAACC CTGTCTCTAC TAAAAATAAA AAATTAGCTG GGTGCGGTGG       653

CAGGCACCTG TAATCCCAGC TACTCAGGAG GCTGAGGCAG GAGAATCACT TGACCCAGGA       713

GGTGGAGGTT ACAGTGAGCC AGATCTCACC ACTGCACTGT AGCCTGGCAA CAGAGCAAGA       773

CTCCATGAAA AAAAAAAAAA AAAAA                                             798

(2) ANGABEN ZU SEQ ID NO: 2:

    (i) SEQUENZKENNZEICHEN:
        (A) LÄNGE: 171 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 2:

Ala Arg Glu Lys Arg Lys Pro Gln Gln Pro Gln Arg Arg Pro Ala Gly

1                   5                   10                  15

Gly Thr Gly Gln Arg Arg Gly Ser Gly Tyr Ser Pro Ser Ala Asp Gln
            20                  25                  30

Gln Gly Ala Gln Asp Arg Glu Glu Glu Ala Ala Ala Ala Pro Ala Pro
        35                  40                  45

Thr Ser Ser Gly His Arg Thr Glu Lys Arg Lys Arg Leu Gln Leu Gln
    50                  55                  60

Cys Gln Pro Ala Gly Gly Thr Gly Gln Arg Arg Gly Ser Gly Gln Gly
65                  70                  75                  80

Pro Ser Ala Arg Pro Ala Ala Phe Thr Gly Gln Arg Arg Gly Ser Arg
                85                  90                  95

Ser Ser Pro Ser Ala Asp Gln Gln Arg Ala Gln Asp Arg Glu Glu Glu
            100                 105                 110

Ala Ala Arg Pro Gln Arg Arg Pro Ala Ala Gly Thr Gly Gln Arg Arg
        115                 120                 125

Gly Ser Ala Ala Ala Pro Val Pro Thr Ser Ser Gly Thr Gly Gln Arg
    130                 135                 140

Arg Gly Ser Ala Ala Ala Pro Ala Pro Thr Ser Ser Gly Thr Gly Gln
145                 150                 155                 160

Arg Arg Gly Ser Glu Glu Met Glu Glu Glu Gly
                165                 170

(2) ANGABEN ZU SEQ ID NO: 3:

     (i) SEQUENZKENNZEICHEN:
          (A) LÄNGE: 854 Basenpaare
          (B) ART: Nucleotid
          (C) STRANGFORM: Doppelstrang
          (D) TOPOLOGIE: linear

    (ii) ART DES MOLEKÜLS: cDNA

    (vi) URSPRÜNLICHE HERKUNFT:
          (A) ORGANISMUS: not known
          (G) ZELLTYP: Kidney tissue (adult)

   (vii) UNMITTELBARE HERKUNFT:
          (A) BIBLIOTHEK: human kidney cDNA, Clontech, Palo Alto (2
               MAX1 (5'-Strech)
          (B) CLON(E): p B1.2 (screening with p B1 (EcoRI/HindII)

    (ix) MERKMAL:
          (A) NAME/SCHLÜSSEL: CDS
          (B) LAGE:577..854

    (xi) SEQUENZBESCHREIBUNG: SEQ ID NO: 3:
   ACGCCTTTGG TGGTTGGCGC CCTCCAGGTC CCCCTCCCTC CAAGTCCAGT AAGAAGTTGG      60
   GCCAAGCTGG AAGGGATTGA CCGGTTGCTT GTCTGGAGAT GGAGCCACTA CAGGCGGGCC     120
   TGGGCGCCTG GAGTCGGGCA TGAAAGAAAA TAGCGCCTCA TCGCTCTTAT CTAAGCCCAG 180
   AGGTCCAGGG GCAACCTGAG GGCCCTGAAG TCTTTCAATA CCATGTACCA GCTTGACTTC 240
   TCTACATGTC CCTTGGATTG CCCAAAAGCA AAATCTCAGA GGGGCCAAGG ACCTTCTAGG 300
   GGGGTCGTCT GCTCTGTCTG GACACACAAG AACCATCATG CGTGTGAGAG CCTTTGGCAT 360
   GAGAGACACA AAGCCACAGG GAAACACATG GTGTGGAGAC TGTGCTGAGA GCAGAGATGC 420
   AGAGATGCAG AGACAGCAGA GATGCAGAGA CAGCAGAGAT GCAGAGACAG CAGAGAGTGG 480
   ACGAACGAGG AGGGTGTGGG GTGGCTGGGG ATGGCGTGAG GTCCAGGGAA CAGGAGGGGC 540
   TCTCGTGCAC CAGCGTGGGT GGAGAAATCA AACAGG ATG AAG CCT CGA CAG AAA 594
   Met Lys Pro Arg Gln Lys
   175
   GAA CAG GAC ACG AGA CTG AGG AAG CTC CGA GAG AGC AGC GAG GGA GAC 642
   Glu Gln Asp Thr Arg Leu Arg Lys Leu Arg Glu Ser Ser Glu Gly Asp
   180 185 190
   CAG TGG CTG GAA AAT GAG AAA ACA AAG CCA CTG AGG CCC CAG CAG CAG 690
   Gln Trp Leu Glu Asn Glu Lys Thr Lys Pro Leu Arg Pro Gln Gln Gln
   195 200 205
   CCC CAG CGC CGA CCA GCA GGG GGC ACA GGA CAG AGA AGA GGA AGC GGC 738
   Pro Gln Arg Arg Pro Ala Gly Gly Thr Gly Gln Arg Arg Gly Ser Gly
   210 215 220 225
   AGC AGC CCC AGC GCC GAC CAG CAG CGG GCA CAG GAC AGA GAA GAG GAA 786
   Ser Ser Pro Ser Ala Asp Gln Gln Arg Ala Gln Asp Arg Glu Glu Glu
   230 235 240
   GCC GCA GCA GCT CCA GTG CCT AAC CAG CAG CGG GCA CAG GAC AGA GAA 834
   Ala Ala Ala Ala Pro Val Pro Asn Gln Gln Arg Ala Gln Asp Arg Glu
   245 250 255
   GAG GAA GCC GCA GCA GCC CC 854
   Glu Glu Ala Ala Ala Ala
   260

(2) ANGABEN ZU SEQ ID NO: 4:

(i) SEQUENZKENNZEICHEN:
(A) LÄNGE: 92 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 4:

Met Lys Pro Arg Gln Lys Glu Gln Asp Thr Arg Leu Arg Lys Leu Arg
1               5                   10                  15

Glu Ser Ser Glu Gly Asp Gln Trp Leu Glu Asn Glu Lys Thr Lys Pro
            20                  25                  30

Leu Arg Pro Gln Gln Gln Pro Gln Arg Arg Pro Ala Gly Gly Thr Gly
        35                  40                  45

Gln Arg Arg Gly Ser Gly Ser Ser Pro Ser Ala Asp Gln Gln Arg Ala
    50                  55                  60

Gln Asp Arg Glu Glu Glu Ala Ala Ala Ala Pro Val Pro Asn Gln Gln

65                    70                    75                    80

Arg Ala Gln Asp Arg Glu Glu Glu Ala Ala Ala Ala
                85                    90

(2) ANGABEN ZU SEQ ID NO: 5:

      (i) SEQUENZKENNZEICHEN:
           (A) LÄNGE: 2578 Basenpaare
           (B) ART: Nucleotid
           (C) STRANGFORM: Doppelstrang
           (D) TOPOLOGIE: linear

     (ii) ART DES MOLEKÜLS: Genom-DNA

    (iii) HYPOTHETISCH: NEIN

     (iv) ANTISENSE: NEIN

     (vi) URSPRÜNLICHE HERKUNFT:
           (A) ORGANISMUS: not known
           (C) INDIVIDUUM/ISOLAT: isolated by screeing with p B1
                (EcoRI/HindII)
           (F) GEWEBETYP: human
           (G) ZELLTYP: peripheral lymphocytes

    (vii) UNMITTELBARE HERKUNFT:
           (A) BIBLIOTHEK: human cosmid-gen-library, Clontech, Palo Alto
           (B) CLON(E): p WE-B1 (isolated by screening with p B1
                (EcoRI/HindII)

     (xi) SEQUENZBESCHREIBUNG: SEQ ID NO: 5:

GTGGGGCTGG CCAACAGTGC CAATGTCAAG ACCCACAGCC ATGATAGAAT TAAGTTAGTG      60

TTTAAACAGA CATGCCCCGG AGTCTGGACT GATAGAGGGG AGTGCTCTGT GCAGAAGCAG     120

CTGTCGCCAT CATCCGGCCA CCACCGCCAG GTAGCAACCA CAGGGGAGGA AAAGTGTGCA     180

GATTCCAGGG CCTGGGAGAG ACCAGCAGGA CCTGCTGCAG GGCTGGAAGT GTGGAGAAGA     240

GAAGGAGAAA GTGTGACCTC CAGGATCTGA CTTCAGCCAC GGCATGGGGC ACCCTTCACT     300

GTGGGGTGTT GAAGGAAGAC CCAGAGGGCA GGGAGGAGGC GTGTGGTGTC CCTGCAGGCA     360

CCAGATGACA GCGGGGCTGG GTATAAAACA TGGTGCTGCC AGCAAGGAGC TGGAACGTGG     420

AGCCACAGCG AAGGGAGGAG GGTGCGCACG AGGCCTATGA CAGCTCATGC ACGGCTGGAG     480

TCTGGGTAAG TGGAGGGGCA GCAAGGGTGG GGCTGATGGT CCAGACAGAG CAGACGACCC     540

TTGCTGTCCA CTATGGAAGG ACACACAAGA ACCACTATGC GTGTGAGAGC CTTTGCGATG     600

AGAGACACAA AGCCACAGGG AAACACATGG TGTGGAGACT GTGCTGAGAG CAGAGATGCA     660

GAGATGCAGA GACAGCAGAG ATGCAGAGAC AGCAGAGAGT GGACGAACGA GGAGGGTGTG     720

GGGTGGCTGG GGATGGCGTG AGGTCCAGGG AACAGGAGGG GCTCTCGTGC ACCAGCGTGG     780

GTGGAGAAAT CAAACAGGAT GAAGCCTCGA CAGAAAGAAC AGGACACGAG ACTGAGGAAG     840

CTCCGAGAGA GCAGCGAGGG AGACCAGTGG CTGGAAAATG AGAAAACAAA GCCACTGAGG     900

CCCCAGCAGC AGCCCCAGTG CCAACCAGCA GGGGGCACAG GACACAGAAG AGGAAGCGGC 960
AGCAGCCCCA GCGCCGACCA GCAGGGGGCA CAGGACAGAG AAGAGGAAGC GGCAGCAGCC 1020
CCAGCGCCGA CCAGCAGGGG GCACAGGACA GAGAAGAGGA AGCCGCAGCA GCCCCAGCGC 1080
CGACCAGCAG GGGGCACAGG ACAGAGAAGA GGAAGCCGCA GCAGCTCCAG CGCCGACCAG 1140
CAGGGGGCAC AGGACAGAGA AGAGGAAGCG GCTGCAGCCC CAGCGCCGAC TAGCAGGGGG 1200
CACAGGACAG AGAAGAGGAA GCGGCAGCAG CCCCAGCGCC GACCAGCAGG GGGCACAGGA 1260
CAGAGAAGAG GAAGCGGCAG CAGCCCCAGC GCCGACCAGC AGGGGGCACA GGACAGAGAA 1320
GAGGAAGCGG CAGCAGCCCC AGCGCCGACC AGCAGGGGGC ACAGGACAGA GAAGAGGAAG 1380
CGGCAGCAGC CCCAGCGCCG ACCAGCAGGG GGCACAGCAC GAGAGAAGAG GAAGCGGCAG 1440
CAGCCCCAGC GCCGACCAGC AGGGGGCACA GGACAGAGAA GAGGAAGCGG CTGCAGCCCC 1500
AGCGCCGACC AGCAGGGGGC ACAGGACAGA GAAGAGGAAG CCGCAGCAGC CCCAGTGCCG 1560
ACCAGCAGCG GGCACAGGAC AGAGAAGAGG AAGCGGCTGC AGCTCCAGTG CCAACCAGCA 1620
GGGGGCACAG GACAGAGAAG AGGAAGCGGC TGCAGCCCCA GTGCCAACCA GCAGGGGGCA 1680
CAGGACAGAG AAGAGGAAGC GGCAGCAGCC CCAGCGCCGA CCAGCAGGGG GCACAGGACA 1740
GAGAAGAGGA AGCGGCAGCA GCCCCAGCGC CGACCAGCAG GGGGCACAGG ACAGAGAAGA 1800
GGAAGCGGCA GCAGCCCCAG TGCCGACCAG CAGCGGGCAC AGGACAGAGA AGAGGAAGCC 1860
GCAGCAGCCC CAGCGCCGAC CAGCAGCGGG CACAGGACAG AGAAGAGGAA GCGCAGCAGC 1920
CCCAGCGCCG ACCAGCAGCG GGCACAGGAC AGAAAAGAGG AAGTGAGGAA ATGGAAGAAG 1980
AGGGTTGACC TGAGCTGAAG GACATGAACT TCCACGTGAA ATAGCCCCAG GGCCGGGCAC 2040
AGTGGCGCAC GCCTGTAATC CCAGCACTTT GGGAGTCTGA GGCAGGAGGA TTCCTTGAGC 2100
CCAGGAGTTC GAGACCAGCC TGGGCAACAC AGTGAGACCC ACCCCCTCCA TCTCTACAAA 2160
AAATACAAAA ATTAGCCAGA CACAGTGGCT CACACCTGTA GTCCCAGCTG CCCCAGAGTC 2220
TAAAGCAGGA GGCTCACTGG AGCACAGGAG TTCGAGGCTG CCGTGAGCTA CGATTGCACC 2280
ACTGCACTCC AGCCTGGGCG ACAGGATGAG ACTGTGTCTC AAAAGAAGAA AAAAGGAAAA 2340
ACTAATAGTT AAAAGTAAAT AAATAGTCCC GGTTTCCAGC AGAATAAAAG GAAGGTCAGC 2400
AGAAAGGCCA GGACGGCCTG AAGGAACTTC AGGGCTGGGA CTGTGGGGTG GAGCCGTCCC 2460
TTCCGGATGC CGAGCGAACG GACCTCCCCG CAAAAGCACA GGGGTGAGGG GATGAAGTCA 2520
TGTTCCACAC ACAGGATTTA GACACGGCTC ATGGCCCATG CACCCGAAAC CAGGATGT 2578

## Beschreibung

Die vorliegende Erfindung betrifft die Isolierung und Charakterisierung von Aminosäuren- und Nukleotidsequenzen eines neuen Mitglieds der Muzingenfamilie. Insbesondere betrifft die Erfindung die Bereitstellung von Reagenzien für die Diagnostik von Patienten und für die Vakzinierung zur Behandlung bestimmter Erkrankungen durch Stimulierung der Immunabwehr.

Die Epithelien des respiratorischen, reproduktiven und gastrointestinalen Traktes höherer Organismen sind mit einem protektiven Sekret, Mucus genannt, ausgekleidet. Dieses Mucus-Gel besteht zu 95 % aus Wasser und zu ungefähr 5 % aus Muzinen (Neutra und Forstner, 1987). Muzine sind Glykoproteine mit zwei spezifischen Merkmalen: Erstens bestehen mindestens 50 % ihres Molekulargewichtes aus Oligosacchariden, die O-glykosidisch an Threonin- und Serinresten des Proteingerüstes gebunden sind, und zweitens besteht dieser stark glykosylierte Bereich aus repetitiven Sequenzeinheiten. Die Muzine lassen sich in zwei Gruppen einteilen, zum einen in die sekretorischen Muzine, die über intermolekulare Disulfidbrücken als Oligomere vorliegen, und zum anderen in die membranständigen Muzine, die über einen hydrophoben Bereich in der Plasmamembran verankert sind (Gum, 1992; Metzgar et al., 1993; Strous und Dekker, 1992).

Seit 1987 wurden DNS Sequenzen für mittlerweile sieben Mitglieder der Muzinfamilie aus humanen Genbanken isoliert und charakterisiert. Der Sequenzvergleich erbrachte innerhalb der verschiedenen Muzine keine signifikante Homologie, weder in ihrer Nukleotid- noch in ihrer Aminosäurenzusammensetzung. Allen Muzinen gemeinsam ist aber ein zentraler Bereich, der die O-Glykane trägt und aus aufeinanderfolgenden, repetitiven Nukleotidsequenzen und Aminosäurenmotiven besteht.

Veränderungen in Expression und posttranslationaler Modifikation der Muzine sind mit verschieden Krankheiten, wie Karzinomen, zystischer Fibrose, Colitis ulcerosa und Enteritis regionalis Crohn assoziiert und lassen Muzine zum Gegenstand klinischer Forschungen werden. Antikörper, die Muzinepitope erkennen (CA 19-9, CA 125 und M26), werden als Marker in der Diagnostik eingesetzt (Magnani et al., 1984; Metzgar et al., 1984; Linsley et al., 1988).

Es ist ein Ziel vieler Forscher, neue Informationen über Polypeptid- und Nukleinsäuresequenzen von Muzinen zu erhalten. Da die Anreinigung der hochmolekularen Muzin-Glykoproteine, trotz Fortschritte in der Proteinchemie, oft erfolglos blieb und auch die Anwendung der rekombinanten DNA Technologie bei den hochrepetitiven Nukleotidsequenzen problematisch ist, besteht ein großer Bedarf an zusätzlichen Muzinen, die Fortschritte in Diagnostik und Behandlung von Patienten ermöglichen würden.

Der vorliegenden Erfindung lag somit die Aufgabe zugrunde, weitere Muzine zu finden, um insgesamt die diagnostische Wertigkeit der Muzine zu erhöhen und damit einen wesentlichen Beitrag zur Tumordiagnostik zu leisten.

Die Lösung dieser Aufgabe erfolgt durch die Bereitstellung der in den Patentansprüchen beschriebenen Ausführungsform, insbesondere in der Bereitstellung eines neuen Muzins bzw. Muzigens.

Die hier behandelte Erfindung stellt Nukleotidsequenzen, die für ein neues humanes Muzin kodieren, zur Verfügung. Die Nukleotidsequenzen liegen als genomische DNA und cDNA vor, die in zirkuläre Plasmid-Vektoren integriert sind. Mit diesen Konstrukten, die Muzin-Sequenzen enthalten, wurden bakterielle Wirtsorganismen (E. coli) stabil transformiert, die es nun ermöglichen, die Muzinsequenzen in unbegrenzten Mengen zu amplifizieren.

Durch Expression der kodierenden Sequenz in einem prokaryotischen Expressionssystem ist die Produktion und Anreinigung des rekombinanten Polypeptids in großen Mengen ohne Verunreinigungen durch andere humane Glykoproteine möglich. Der bakterielle Wirt erlaubt eine unproblematische Kultivierung im großen Maßstab. Für die Isolierung des Polypeptids aus den Zellysaten kann eine präparative Affinitätschromatographie mittels des mAk B1 oder Maltose durchgeführt werden, da das Polypeptid als Fusionsprotein mit dem Maltose-Bindungsprotein MBP synthetisiert wird. Die Expression des Polypeptids ist durch Induktion des bakteriellen Promotors mit IPTG gezielt zu beeinflussen.

Dieses definierte Polypeptid erlaubt die Etablierung eines ELISA-Systems zum Screenen von Patientenseren und kann für die Generierung weiterer Antikörper benutzt werden.

Durch Kopplung des Antikörpers mit alkalischer Phosphatase oder Meerrettich-Peroxidase ist die Entwicklung eines Sandwich-ELISA Systems möglich, das es erlaubt, durch Bindung anderer definierter Antikörper an die Festphase weitere antigene Determinanten auf dem nativen humanen MUC8 Glykoprotein nachzuweisen.

Mit Hilfe der neuen Erfindung können so diagnostische Kits entwickelt werden, die einen routinemäßigen Einsatz in der Klinik ermöglichen.

Da in Southern-Blot Analysen die Lokalisation des MUC8 Gens auf dem humanen Chromosom Nr. 7 gezeigt werden konnte, eignen sich markierte DNA Sonden (radioaktiv oder nicht-radioaktiv mit Biotin) als Genmarker zur Identifizierung von benachbarten Genen oder den entsprechenden Mutationen (Deletionen, Translokationen und Insertionen). Da das MUC8 Gen hochpolymorph ist, wie durch Hybridisierung mit DNA Proben von verschiedenen Patienten gezeigt werden konnte, ist es möglich MUC8-DNA als Sonde in Fingerprint-Analysen zur Identifizierung von Verwandtschaftsbeziehungen einzusetzen.

Ein weiterer Aspekt stellt die Verwendung von antisense-RNA bzw. antisense-Oligonukleotiden dar, die es ermöglicht die Translation der MUC8 mRNA spezifisch zu blockieren. Dies könnte zu einer besseren Prognose des Tumors führen, da Muzinexpression von Tumorzellen zu einer erhöhten Metastasierungsrate im Tiermodell führt (Bresalier et al., 1991). Eine weitere Anwendungsmöglichkeit stellen Vakzinierungsversuche dar. Für MUC1 Peptide konnte gezeigt werden, daß Muzin-Epitope MHC-unrestringiert von zytotoxischen T Zellen erkannt werden und somit eine tumorspezifische Immunantwort auslösen können (Hilkens et al., 1992; Barnd et al., 1989; Ioannides et al., 1993).

In immunhistochemischen Untersuchungen von Kryostatschnitten verschiedener benigner und maligner Gewebe konnte das Expressionsmuster des B1 Antigens im Menschen bestimmt werden. Aus diesen Daten kann der mögliche Einsatzort von diagnostischen Systemen zum Nachweis des MUC8 Muzins abgeleitet werden.

Im Rahmen der hier beschriebenen Erfindung wurden Nukleotidsequenzen, die für das humane Muzin MUC8 kodieren, isoliert. Ein Teil der repetitiven Nukleotidsequenz wurde exprimiert, um größere Mengen des Polypeptids mit seinen biologischen und immunologischen Eigenschaften isolieren und untersuchen zu können.

Die dazu verwendete DNA wurde durch Immunscreening einer IZAP cDNA Genbank, hergestellt aus mRNA der humanen Kolonkarzinom-Zellinie T84, isoliert. Der Klon wurde pB1 genannt und umfaßt 798 bp. Die cDNA enthält einen 3'-untranslatierten Anteil von 282 bp, der aus Alu-Repeats besteht, ein Polyadenylierungssignal enthält und mit einem poly(A)-Schwanz endet. Die kodierende Sequenz von 517 Nukleotiden besteht aus repetitiven Einheiten von 53 bp und endet mit einem Opal-Stopkodon. Ein EcoRI/HindII Fragment dieser cDNA, das den kodierenden, repetitiven Bereich umfaßt, wurde als Sonde in Northern-Blot Analysen eingesetzt. Drei mRNA Spezies von 4,8 kb, 1,9 kb und 1,35 kb hybridisierten in humanen Tumorzellinien, von denen die 4,8 kb Bande das stärkste Signal darstellte. In Southern-Blot Analysen, die mit EcoRI und MspI verdauten, genomischen, humanen DNA Proben angefertigt wurden, konnte mit dem EcoRI/HindII Fragment als Sonde ein Restriktionsfragmentlängenpolymorphismus festgestellt werden, der bei den MspI verdauten Proben am deutlichsten hervortrat. Um weitere Sequenzinformation zu erhalten, wurde eine humane Nieren cDNA Genbank in dem Phagen IMAX1 und eine genomische Genbank in dem Kosmid-Vektor pWE-15 mit dem EcoRI/HindII Fragment gescreent. Aus der IMAX1 Genbank konnte ein cDNA Fragment von 854 bp Länge isoliert werden. Dieser Klon pB1.2 enthält einen 5'-untranslatierten Bereich von 576 bp, dem nach einem potentiellen Startkodon eine kodierende Sequenz von 278 bp folgt. Im Screening der genomischen DNA Genbank konnte ein positiver Klon pWE-B1 isoliert werden, dessen inserierte DNA mit verschiedenen Restriktionsenzymen geschnitten wurde und anschließend im Southern-Blot analysiert wurde. Ein BglII/BamHI Fragment von 5,9 kb Länge hybridisierte mit beiden B1 cDNA Klonen und wurde zur weiteren Charakterisierung in pBSK(+) subkloniert. Die genomische DNA des resultierenden Klons pB1-Cos wurde vollständig sequenziert und die Nukleotidsequenz mit den beiden cDNA Klonen verglichen. Der Homologie-vergleich ergab, daß der pB1-Cos Klon ein Exon von 1482 bp enthält, dessen 5'-Bereich mit Teilen des pB1.2 Klons identisch ist, während der 3'-Bereich mit Teilen des pB1 Klons übereinstimmt. Der mittlere Bereich des Exons, der nicht durch cDNA Nukleotidsequenzen repräsentiert wird, besteht, wie Partialverdau-Analysen mit dem Restriktionsenzym Earl ergaben, aus zusätzlichen repetitiven Einheiten. Insgesamt enthält das Exon des pB1-Cos Klons 19 Earl Subfragmente von 53 bp Länge. Der Genort des MUC8 Gens konnte im Southern-Blot mit DNA aus somatischen Zellhybriden auf Chromosom 7 festgelegt werden.

DNA Fragmente, die für das MUC8 Muzin kodieren, können in eukaryotische Expressionsvektoren inseriert werden und zur Transformation (stabil oder transient) von Säugerzellen, insbesondere humanen Zellen, verwendet werden. Da die neue Generation von Vektoren üblicherweise Enhancer- und Promotorsequenzen bereit stellt und eine Selektion sowohl im prokaryotischen als auch eukaryotischen Systemen ermöglicht, ist eine einfache Handhabung der eukaryotischen MUC8 Expression gewährleistet. Dadurch kann ein ko- und posttranslational-modifiziertes Polypeptid, das dem nativen MUC8 Muzin entsprechen dürfte, isoliert und analysiert werden. Dieses Konstrukt kann in, mit Epstein-Barr Virus immortalisierten, humanen B-Lymphozyten stabil eingeführt werden, um eine Präsentierung von MUC8 Peptidfragmenten mit MHC-Klasse I und II zu ermöglichen. Durch Inkubation der transfizierten Zellen mit Inhibitoren der posttranslationalen O-Glykosylierung kann die Expression von tumorassoziierten Epitopen noch gesteigert werden (Bu et al., 1993). MHC-unrestringierte Erkennung von Muzinepitope durch zytotoxische T-Zellen, hervorgerufen durch die repetitive Struktur der Muzine, konnte wiederholt für das MUC1 Muzin gezeigt werden. Zugrunde liegt die Idee, die Transfektanten, die große Mengen MUC8 exprimieren, in Patienten zurückzuführen, um eine spezifische tumorassoziierte Immunantwort durch Antikörper- oder CTL-Erkennung hervorzurufen.

Ein anderer Aspekt ist der Nachweis von Tumoren oder Entzündungen in Patienten mittels Detektion von Muzinantigenen in Geweben oder Seren. Immunologische Methoden, wie immunhistochemische Färbung von Gewebeschnitten, ELISA oder Western-Blot mit Patientenseren, sind mit der Hybridomzellinie B1, die es erlaubt, Antikörper in nahezu unbegrenzter Menge aus dem Kulturüberstand zu isolieren, möglich. Das Hybridom wurde durch Immunisierung einer Balb/c Maus mit einer hochmolekularen Proteinfraktion eines Mz-Sto-1 Zellextraktes und anschließender Fusionierung der Milzzellen der immunisierten Maus mit immortalisierten Myelomzellen gewonnen (Herbsthofer, 1990). Die Magenadenokarzinomzellinie Mz-Sto-1 wurde von einem Patienten, der an Siegelringzellkarzinom erkrankt war, etabliert (Dippold et al., 1987). In der Western-Blot Analyse konnten in Seren von Pankreaskarzinom-Patienten, die erhöhte CA19-9 Werte aufwiessen, gestiegene MUC8-Level im Vergleich mit Seren von Kolonkarzinom-Patienten und gesunden Spendern nachgewiesen werden. Dies gibt erste Hinweise für die Etablierung von MUC8 als Tumormarker in Patientenseren.

Durch Expression der pB1 cDNA in dem Vektor pMAL-c, der es ermöglicht, nach Induktion mit IPTG, ein Fusionsprotein mit N-terminalen Maltose-Bindungsproteinanteil zu exprimieren, steht ein Polypeptid zur Verfügung, das durch den Antikörper B1 erkannt wird und somit die antigene Determinante enthalten muß. Computeranalysen ergaben, daß das Hexamer QDREEE (Ein-Buchstaben-Kode) das wahrscheinlichste Epitop darstellt. Dieses Peptid ist in jeder repetitiver Einheit, die sich auf Aminosäurenebene aus 53 Resten zusammensetzt, kodiert durch 159 bp auf Nukleotidebene, enthalten. Deshalb kann es zur spezifischen Blockierung einer MUC8 Antigen-Erkennung in immunologischen Verfahren verwendet werden.

Die MUC8 Polypeptide, die nach prokaryotischer oder eukaryotischer Expression isoliert werden können, können als Schutzfaktoren bei verschiedenen Erkrankungen (z.B.: Ulcera) direkt angewandt werden oder zur Generierung weiterer, MUC8-spezifischer Antikörper verwendet werden. Wenn die Polypeptide größer als 10 kD sind, können sie direkt als Immunogen verwendet werden, ansonsten ist eine Kopplung an Trägerproteine für eine effektive Immunisierung notwendig. Nach Injektion des Peptids in einen Vertebraten (Maus, Ratte, Kaninchen, Ziege oder Schaf), können aus den Tieren MUC8-spezifische Antikörper als polyklonales Antiserum isoliert werden. Für die Herstellung monoklonaler Antikörper muß nach der Methode von Milstein und Köhler, die schon für die Herstellung des mAk B1 erfolgreich verwendet wurde, vorgegangen werden. Aus den Kulturüberständen der Hybridomzellinien können die Antikörper nach entsprechenden, für jede Subklasse optimierten, Protokollen angereinigt werden (siehe Harlow, E. und Lane, D. "Antibodies: A Laboratory Manuel" (1988) Cold Spring Harbor Laboratory, Cold Spring Harbor, New York). Die Polypeptide sowie die Antikörper können modifiziert werden, indem kovalent oder nicht kovalent Substanzen, die eine Detektion erlauben, konjungiert werden. Diese Substanzen können Radionuklide, Enzyme, Kofaktoren, magnetische Partikel, Fluoreszenz- oder Chemilumineszenzfaktoren darstellen. Diese modifizierten Antikörper oder Polypeptide können in verschiedenen immunologischen Techniken zum Nachweis von MUC8 Muzin in biologischen Materialien, wie Gewebebiopsien, Blut, Urin, Serum und anderen Körperflüssigkeiten, eingesetzt werden. Gewebeproben werden üblicherweise als Paraffin- oder Kryostatschnitte auf Objektträgern unter Verwendung eines markierten MUC8 Antikörpers oder eines unmodifizierten Antikörpers, dessen Bindung mit einem zweiten markierten Antikörper nachgewiesen werden muß, untersucht. In ELISA-Systemen und Western-Blot Analysen können Proteine aus Körperflüssigkeiten auf einfache Weise untersucht werden. Es gibt eine Vielzahl kompetitiver und nicht kompetitiver immunologischer Testsysteme, in denen Antigen oder Antikörper an die Festphase gekoppelt sind (Harlow und Lane, 1988).

Für eine Verwendung in der Krebstherapie können die MUC8-spezifischen, möglichst humanisierten Antikörper mit Toxinen oder Radionukliden gekoppelt und den Patienten verabreicht werden, deren Tumorgewebe eine erhöhte MUC8 Expression zeigt, um spezifisch das Tumorgewebe zu schädigen bzw. zu markieren.

### Abkürzungen:

- bp: Basenpaare
- BSA: Rinderserumalbumin
- h: Stunde
- kD: Kilodalton
- MBP: Maltose-Bindungsprotein
- RT: Raumtemperatur

Die folgenden Beispiele erläutern die Erfindung.

### Beispiel 1

### A) Screenen von Phagengenbanken

Eine humane cDNA Expressions-Genbank im Phagen Uni-ZAP XR (Stratagene) wurde aus mRNA der Kolonkarzinom-Zellinie T84 hergestellt. Die Phagen wurden in Soft-Agarose in einer Dichte von 5 x 10⁴ pfu /150 mm Platte plattiert und 3,5 h bei 42 °C inkubiert. Danach wurden die mit IPTG (Isopropyl β-D-thiogalaktopyranosid) gesättigten Nitrozellulosefilter aufgelegt und weitere 3 h bei 37 °C inkubiert. Die Filter wurden abgenommen und, nach Absättigung unspezifischer Bindungen, mit unverdünnten B1 Hybridomüberstand bei RT 1 h inkubiert. Als 2. Antikörper wurde 1:1000 verdünnter anti-Maus, alkalische-Phosphatase gekoppelter, Antikörper (DAKO) verwendet. Die Substratumsetzung erfolgte mit BCIP und NBT. Nach drei Rescreens konnte ein positiver Plaque kloniert werden. Nach in vivo Exzision durch den Helferphagen R408 wurde das Plasmid pBluescript SK(-) mit der inserierten B1 cDNA isoliert.

Eine humane Nieren-cDNA Genbank im Phagen MAX1 (Clontech) wurde mit einer Dichte von 3 x 10⁴ pfu/150 mm Platte plattiert und, nach 4 h Inkubation bei 37 °C, Nitrozellulosefilter aufgelegt. Nach Denaturieren und Neutralisieren werden die Nitrozellulosefilter zur Fixierung der DNA 2 h bei 70 °C gebacken. Die Prähybridisierung sowie die Hybridisierung erfolgte in 5 x Denhardts, 0,5 % SDS, 1 M NaCl, 5 mM EDTA (pH 8), 4 mM NaH₂PO₄, 37 mM Na₂HPO₄, 10 mg/ml Heringssperma-DNA und 50 % Formamid bei 42 °C. Ein EcoRI/HindII Fragment der pB1 cDNA wurde nach der "random-primed" Methode mit ³²P a-dCTP radioaktiv markiert und zur Hybridisierungslösung gegeben. Nach 16 h wurden die Filter bei 65 °C 1 h in 1 x SSC + 0,1 % SDS gewaschen und auf einem Röntgenfilm exponiert. Von vier positiven Klonen blieb nach drei Rescreens ein positiver Plaque, aus dem durch in vivo Exzision mit dem Helferphagen R408 das Plasmid pYEUra3 erhalten wurde, dessen inserierte cDNA nach EcoRI Verdau isoliert werden konnte. Der Klon wurde pB1.2 genannt.

### B) Screenen einer genomischen Kosmid-Genbank

Eine genomische Genbank im Kosmid-Vektor pWE-15 enthält humane DNA aus peripheren Blutlymphozyten und wurde von Prof. Dr. Elisabeth Weiß am Institut für Humangenetik in München zur Verfügung gestellt. Mittels Elektroporation wurden NM544 Bakterien mit der Kosmid-DNA transformiert und 3 x 10⁴ Kolonien/150 mm Platte auf Nitrozellulosefilter ausgespatelt. Nach Inkubation von circa 8 h bei 37°C wird durch Auflegen einer zweiten Nitrozellulosemembran ein Replikafilter hergestellt. Die Bakterien auf dem Replikafilter werden denaturiert und danach neutralisiert, zur Fixierung der DNA 2 h gebacken und mit der EcoRI/HindII Sonde hybridisiert, wie für die Lambda-MAX1 Genbank beschrieben. Ein Klon konnte nach drei Rescreens isoliert werden, dessen Insert von circa 30 kb Länge nach NotI-Verdau isoliert werden konnte. Der Klon wurde pWE-B1 genannt. Nach BamHI/BglII Doppelverdau konnte ein 5,9 kb großes Fragment isoliert werden, das in einen mit BamHI linearisierten pBSK(+) Vektor ligiert wurde. Dieser Klon wurde als pB1-Cos bezeichnet.

### C) DNA Sequenzierung

Alle Sequenzierungen wurden mit doppelsträngiger Plasmid-DNA nach der Dideoxynukleotid-Methode durchgeführt. Für die Sequenzierreaktionen wurden 18-20 mer Oligonukleotide, deren Sequenz aus schon bekannten Nukleotidsequenzen abgeleitet wurde, verwendet. Der T7 Sequencing Kit von Pharmacia wurde genau nach Herstellerempfehlung unter Verwendung von ³⁵S a-dCTP eingesetzt. Die Reaktionen wurden in einem 6 % Polyacrylamidgel aufgetrennt und nach Trocknen des Gels einem Röntgenfilm exponiert.

### D) DNA und RNA Blot-Analysen

Die RNA wurde nach der Guanidiniumisothiocyanat-Methode und anschließender Ultrazentrifugation im Cäsiumchloridgradienten isoliert (Sambrook, J. et al., 1989 Molecular Cloning: A laboratory Manual, Cold Spring Harbor Laboratory, Cold Spring Harbor, New York). Zur Anreinigung von poly(A)⁺ RNA wurde der PolyATtract System III Kit der Firma Promega mit biotinylierten oligo(dT) Primern verwendet. Die so gewonnenen Proben werden in einem 1,2 % Formaldehyd-Agarosegel in 1 x MOPS-Puffer aufgetrennt und mittels Kapillartransfer in 20 x SSC-Puffer auf eine Nylonmembran geblottet. Für die Prähybridisierung und Hybridisierung wird eine Lösung aus 1% BSA, 1 mM EDTA, 250 mM Na₂HPO₄ und 7 % SDS (pH 7) bei 65 °C verwendet. Bei der gleichen Temperatur wird der Blot mit einer Lösung aus 1 mM EDTA, 20 mM Na₂HPO₄ und 1% SDS (pH 7) gewaschen und dann einem Röntgenfilm exponiert.

Die DNA Proben werden aus peripheren Blutlymphozyten, die zuvor über einen Ficoll-Gradienten angereinigt wurden, durch Aufnahme der Zellen in Guanidiniumisothiocyanat-Puffer und anschließender Ethanolpräzipitation gewonnen. Nach Verdau mit entsprechenden Restriktionsenzymen wurden die DNA-Proben in einem 0,8 % Agarosegel in TAE-Puffer aufgetrennt. Die Proben werden im Gel depuriniert, denaturiert und abschließend neutralisiert. Der Transfer auf eine Nylonmembran und die Hybridisierung wird, wie schon für den RNA-Blot beschrieben, durchgeführt.

### F) Expression eines MBP-B1 Fusionsproteins

Das pB1 cDNA Insert von 798 bp wurde in die Stul Schnittstelle des pMAL-c Vektors (Biolabs) in den entsprechenden Leserahmen kloniert. Mit dem Konstrukt pMAL-B1 wurden TB1 Bakterien (Biolabs) transformiert und die resultierenden Transformanten bis zu einer OD₆₀₀ von 0,5 bei 37 °C inkubiert. Für die Zellextraktgewinnung wurde 1 ml der Kultur entnommen und die restliche Kultur mit 0,3 mM IPTG (Endkonz.) induziert. Nach 2 h Inkubation wurden 0,5 ml der induzierten Bakterien entnommen und beide Proben mit SDS-Auftragungspuffer versetzt und in einem 10 % Polyacrylamidgel aufgetrennt (Sambrook et al., 1989).

### G) Immunoblot

Nachdem die im SDS-PAGE aufgetrennten Proteine durch Elektoblotting auf Nitrozellulose transferiert wurden (Transferpuffer: 39 mM Glycin, 38 mM Tris, 0,037 % SDS und 20 % Methanol), wird der Filter nach Absättigen in TBS+ 10 % FCS (für Tumorzellextrakte) oder TBS+1 % BSA (für Bakterienlysate) mit den jeweiligen Antikörpern inkubiert. Als 2. Antikörper wurden immer mit alkalische Phosphatase konjungierte Immunglobuline verwendet und die Substratum-setzung erfolgte mit BCIP und NBT-Tabletten (Sigma).

### H) Immunhistochemische Untersuchen

Von den tiefgefrorenen humanen Geweben wurden mittels eines Kryostaten 10 mm Schnitte angefertigt und mit unverdünnten B1 Hybridomüberstand inkubiert. Als 2. Antikörper wurden anti-Maus Peroxidase-gekoppelte Immunglobuline verwendet. Die Substratumsetzung erfolgt in AEC-Puffer mit H₂O₂.

### I) Isolierung von MUC8 Klonen (cDNA und genomischen Ursprungs)

Da eine biochemische Anreinigung hochmolekularer Glykoproteine, die eine N-terminale Sequenzierung des Proteins erlauben würde, häufig nicht möglich ist, ist eine Aufklärung der Proteinstruktur durch Sequenzierung und Charakterisierung von cDNA Klonen praktikabler.

Der monoklonale Antikörper B1 wurde verwendet, um eine Kolon cDNA Genbank zu screenen. Ein positiver Klon ließ sich nach Screening von 2,4 x 10⁶ rekombinanten Plaques isolieren. Dieser Klon, der pB1 bezeichnet wurde, enthält ein cDNA Insert von 798 bp Länge.

Um zusätzliche Nukleotidsequenzen zu erhalten, wurde eine zweite humane cDNA Genbank, die aus Nieren mRNA hergestellt wurde, unter Verwendung eines radioaktiv markierten pB1 cDNA Fragments gescreent. Von 6,3 x 10⁵ getesteten Plaques ließ sich nach drei Rescreens ein positiver Klon, als pB1.2 bezeichnet, isolieren, dessen inserierte cDNA 854 bp umfaßt.

Da sich beide cDNA Klone durch Anteile repetitiver Sequenzen auszeichnen, aber nicht identisch sind, wurde eine genomische Genbank mit einem pB1 cDNA Fragment gescreent. Es wurden 3 x 10⁵ Kolonien getestet, von denen ein Klon genomische DNA enthält, die mit der cDNA hybridisierte. Das Insert von circa 30 kb wurde mit verschiedenen Restriktionsenzymen geschnitten und in einer Southern-Blot Analyse mit den beiden cDNA Klonen als Sonde nacheinander hybridisiert. Das kleinste, isolierbare Fragment von 5,9 kb Länge wurde in pBluescript subkloniert und der resultierende Klon als pB1-Cos bezeichnet.

### K) Sequenzanalyse der Muzin-Klone

Von allen drei Klonen pB1, pB1.2 und pB1-Cos wurden die Nukleotidsequenzen bestimmt, indem sequenzspezifische Primer für beide DNA-Stränge ermittelt wurden. Der kodierende Bereich der pB1 cDNA besteht aus annähernd 10 repetitiven Einheiten zu je 53 bp. In der pB1.2 cDNA sind drei dieser Repeats am 3'-Ende zu finden. Alle repetitive Einheiten sind durch eine Earl Schnittstelle charakterisiert. Der repetitive Bereich des im Klon pB1-Cos enthaltenen B1-Exons besteht aus 19 Earl Fragmenten. Auf Grund der repetitiven Sequenzeinheiten konnten bis jetzt 225 bp nicht durch Sequenzierung direkt bestimmt werden, da sowohl die Generierung sequenzspezifischer Primer als auch die Zuordnung von Sequenzen aus mit ExoII deletierten Klonen keine sicheren Ergebnisse brachten. Sowohl 321 bp am 5'-Ende des pB1.2 Klones als auch 229 bp am 3'-Ende des pB1 Klones sind nicht durch Sequenzen des pB1-Cos Exons repräsentiert. Dies deutet darauf hin, daß noch mindestens zwei weitere Exons existieren müssen, die diese Sequenzen enthalten. Bestätigt wird diese Annahme da das pB1-Cos Exon von den klassischen AG-GT Sequenzen für Intron/Exon Übergänge begrenzt wird. Da die komplette kodierende Sequenz des MUC8 Muzins aber in diesem einen Exon enthalten ist, kann das Exon zur Klonierung und Expression von Volle-Länge MUC8 verwendet werden. Die Nukleotidsequenz des Exons kodiert für 396 Aminosäuren, die nach Computeranalyse ein Proteingerüst von MW 42178 ergeben. Die abgeleitete Aminosäuresequenz enthält annähernd 6 repetitive Einheiten, bestehend aus jeweils 53 Aminosäuren. Da das im Western-Blot ermittelte Molekulargewicht bei ungefähr 170 kD liegt, müssen eine Oligomerisierung und/oder intensive ko- und posttranslationale Modifikationen gefordert werden, wie sie auch für andere sekretorische Muzine beschrieben sind.

### L) Genomische DNA-Blot Analysen

Genomische DNA Proben, die aus peripheren Blutlymphozyten von humanen Spendern isoliert wurden, werden mit EcoRI oder MspI komplett verdaut und im Southern-Blot mit einem EcoRI/PstI Fragment des pB1 Klons hybridisiert. Alle fünf Proben zeigten einen deutlichen Restriktionsfragmentlängenpolymorphismus, der im MspI Verdau besonders hervortrat. Dort variierten die Banden zwischen 1,5 und 2,9 kb Länge. Die Größe der Fragmente des EcoRI-Verdaus lagen zwischen 10 und 12 kb. In der gleichen Größe hybridisierte auch eine DNA Probe aus somatischen Zellhybriden, die das humane Chromosom 7 enthält.

### M) RNA- und Protein-Blot Analysen

Vier Microgamm poly(A)⁺ RNA aus verschiedenen Tumorzellinien wurden im Northern-Blot mit der 500 bp EcoRI/HindII pB1 cDNA Sonde hybridisiert. Drei mRNA-Spezies von 4,8 kb, 1,9 und 1,35 kb wurden erkannt, von denen die 4,8 Bande die stärkste Hybridisierung zeigte. Ob es sich hierbei um Vorläufer- oder Abbauprodukte der 4,8 Spezies handelt, kann zum gegenwärtigen Zeitpunkt nicht entschieden werden, da die Länge des 5'-untranslatierten Anteils bzw. der Transkriptionsstartpunkt nicht bekannt ist.

Im Western-Blot wird als B1-Antigen ein 170 kD großes Protein unter reduzierenden Bedingungen erkannt. Unter nicht reduzierenden Bedingungen liegt die Bande bei circa 400 kD. Der Anteil der O-Glykosylierung ist unbekannt. N-Glykosylierung kann aufgrund des nicht vorhandenen Konsensusmotivs (Asn-X-Ser/Thr) ausgeschlossen werden.

### Beschreibung der Abbildungen

### Abbildung 1:

Immunoblot von Zellysaten, die aus humanen Tumorzellinien isoliert wurden, mit dem Maus anti-MUC8 mAk B1. Die Proteine wurden in einem 3-10% SDS-Polyacrylamidgel elektrophoretisch nach Molekulargewicht aufgetrennt und auf Nitrozellulose transferiert. Als 1. Antikörper wurde unverdünnter Hybridomüberstand verwendet, als 2. Antikörper anti-Maus Immunoglobuline, konjungiert mit alkalischer Phosphatase. Spur 1: CAMA; Spur 2: Capan-1; Spur 3: Mz-Sto-1; Spur 4: MKN-45; Spur 5: T84; Spur 6: HT-29 MTX;

### Tabelle 3:

Zusammenfassung der immunhistochemischen Daten. Es wurden Kryostatschnitte von verschiedenen benignen und malignen humanen Geweben angefertigt und mit unverdünnten B1 Hybridomüberstand als 1.Antikörper inkubiert. Als 2. Antikörper wurden anti-Maus Immunglobuline konjungiert mit Meerrettich-Peroxidase verwendet.

### Abbildung 2:

Nukleotidsequenz des pB1 cDNA Klons mit abgeleiteter Aminosäuresequenz im Drei-Buchstaben-Kode. Die Sequenz wurde durch Generierung von sequenzspezifischen Primern mittels des T7 Sequencing Kits von Pharmacia nach der Methode von Sanger ermittelt.

### Abbildung 3:

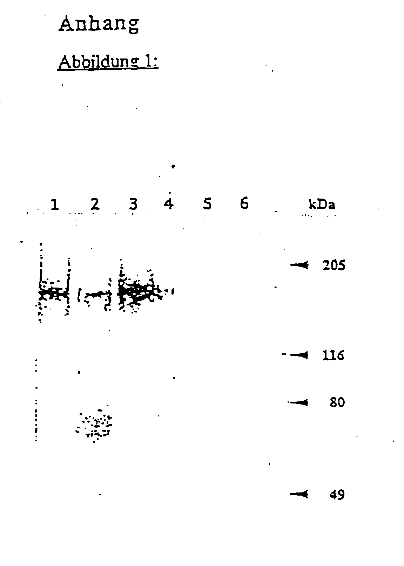

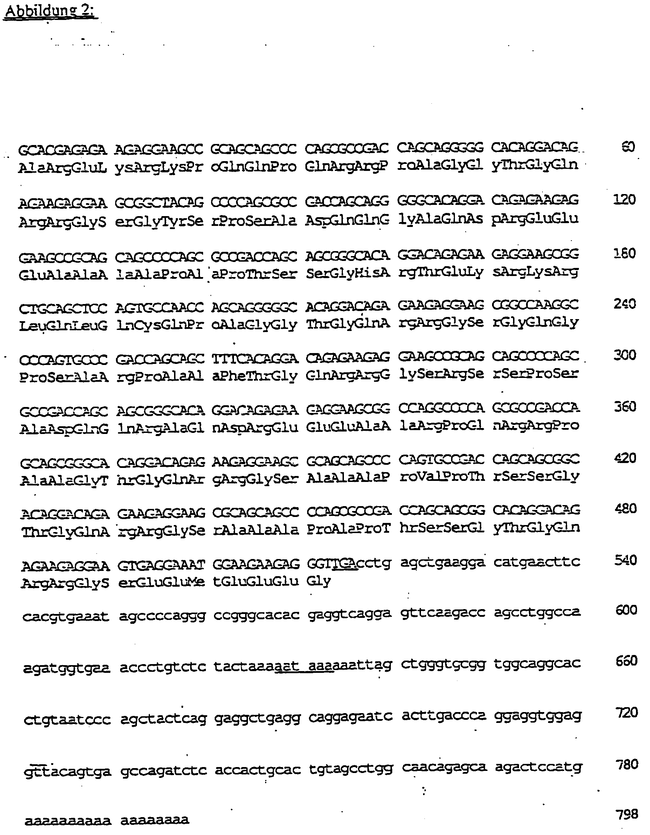

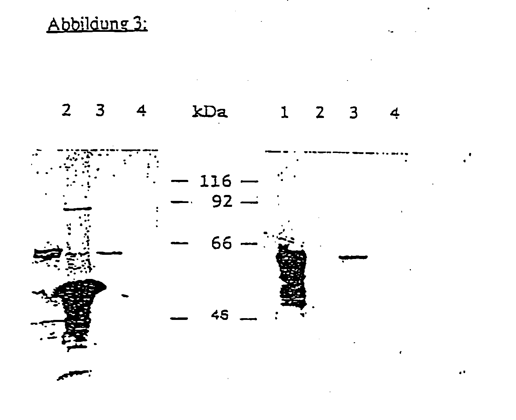

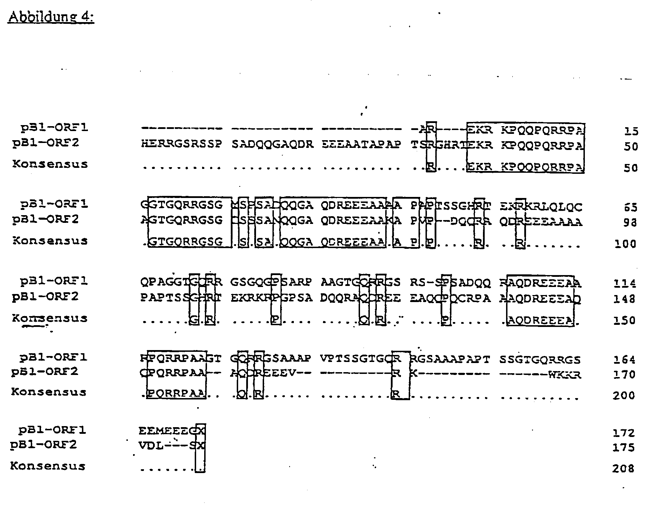

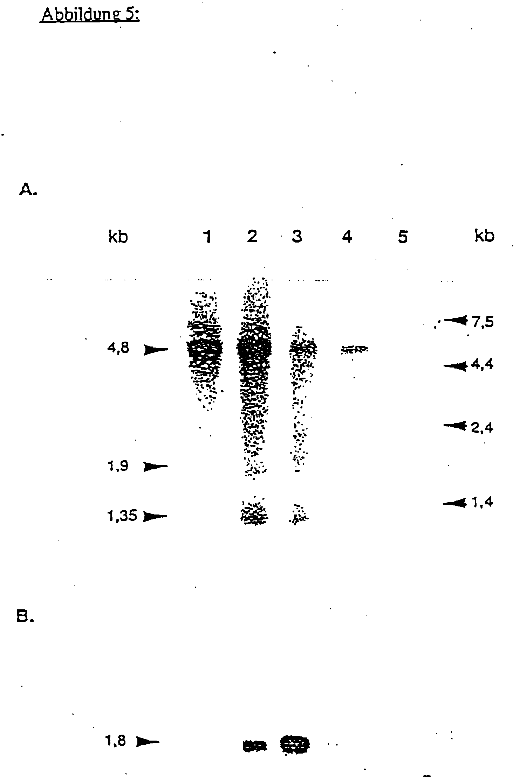

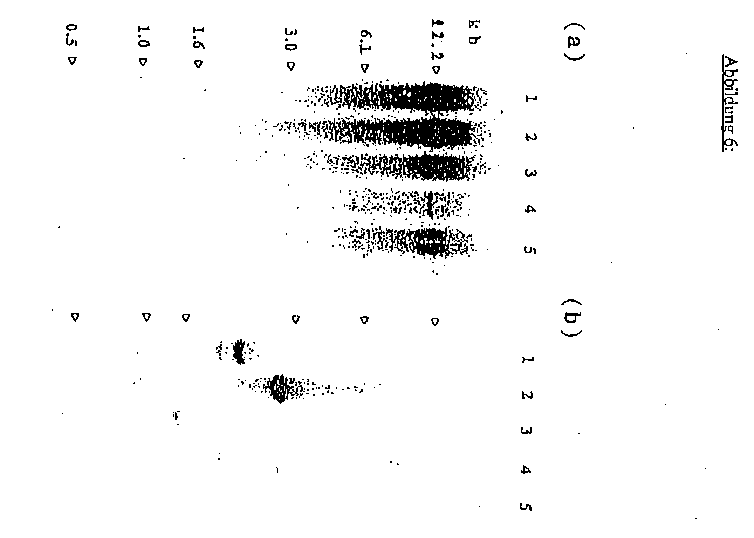

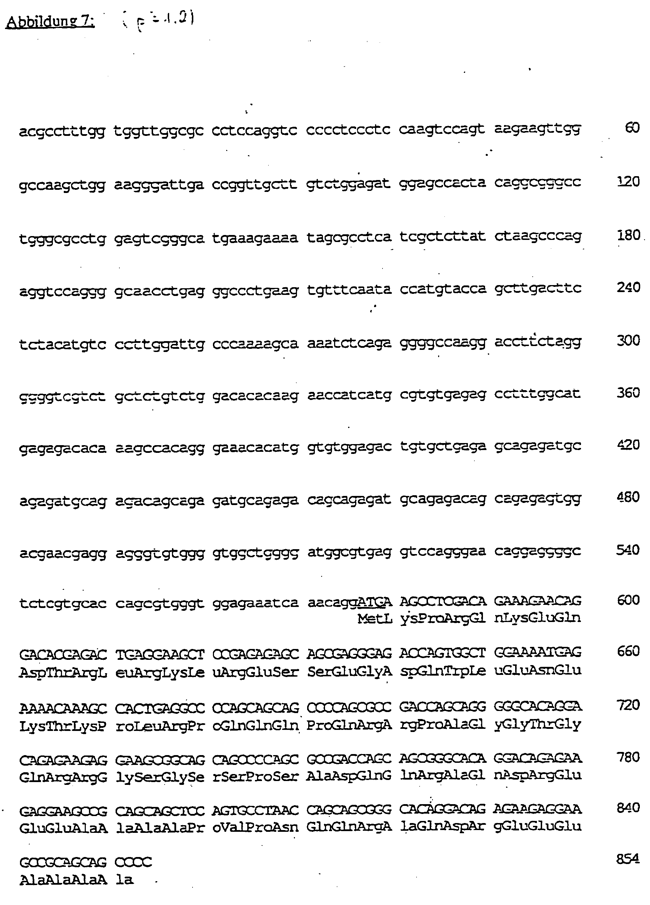

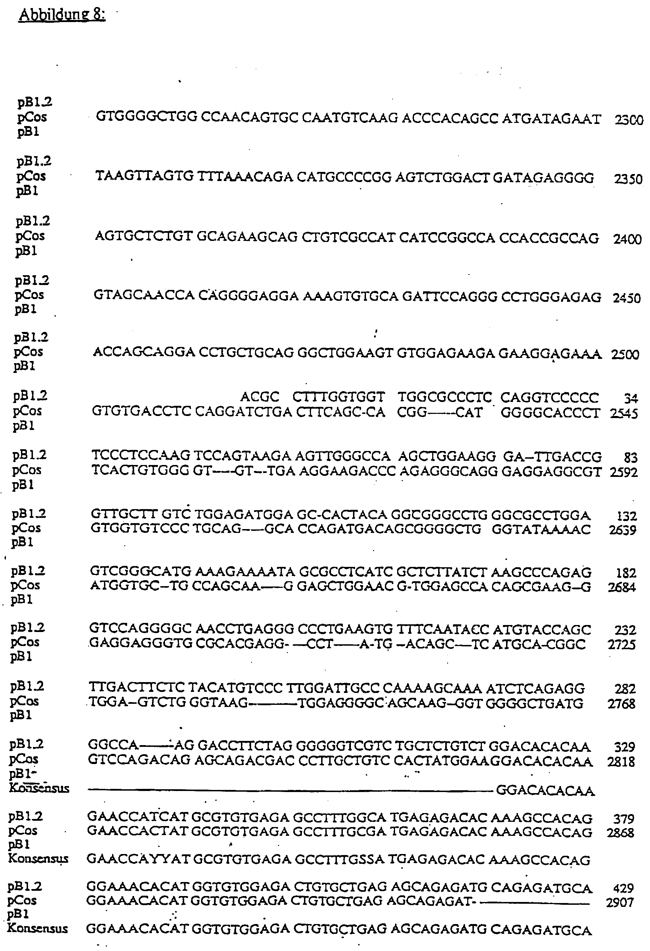

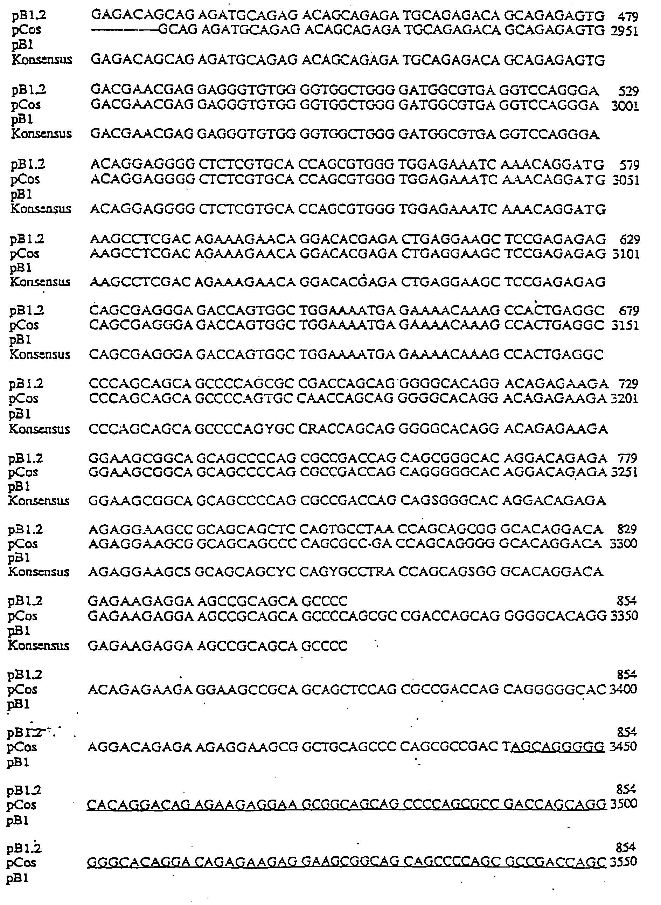

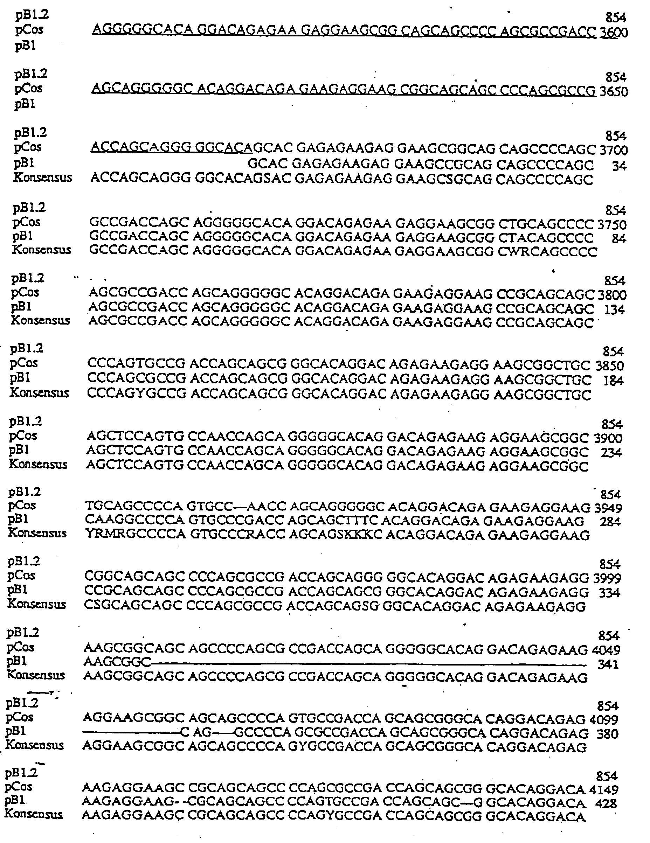

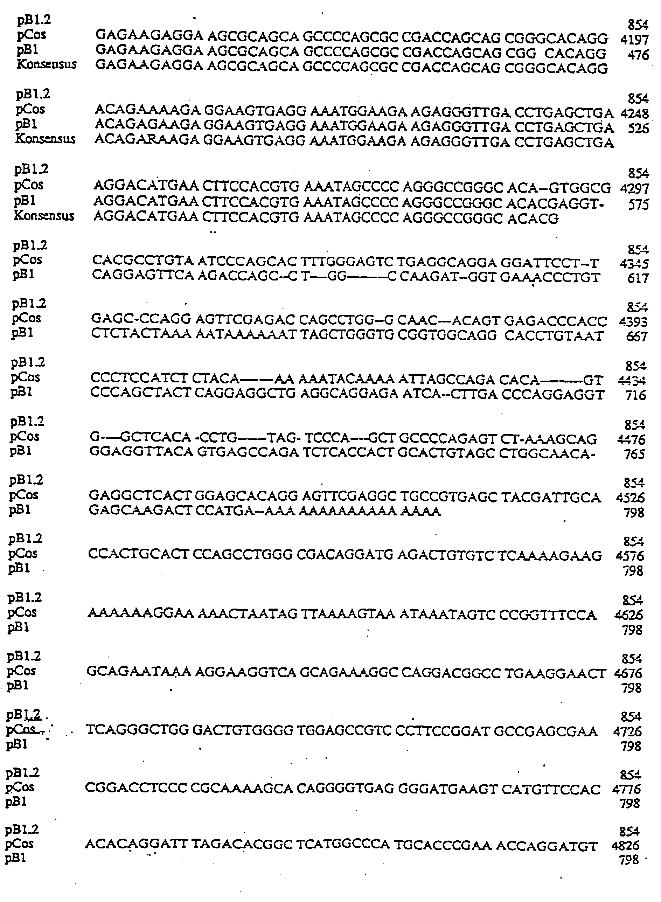

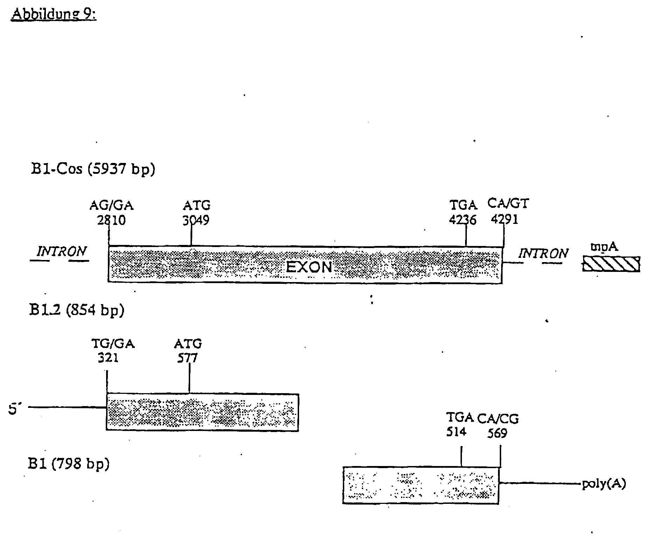

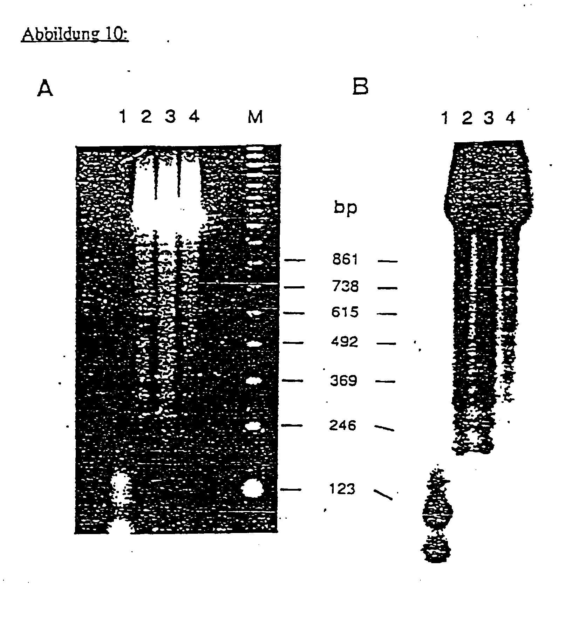

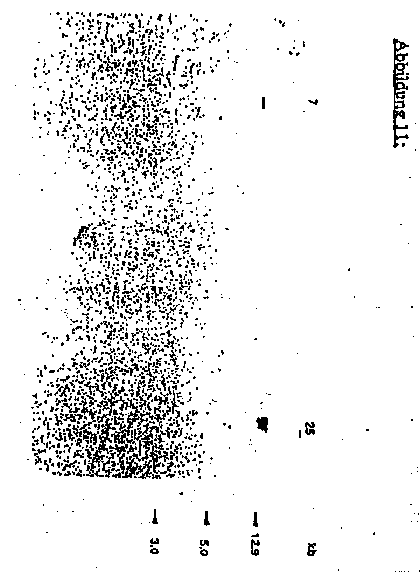

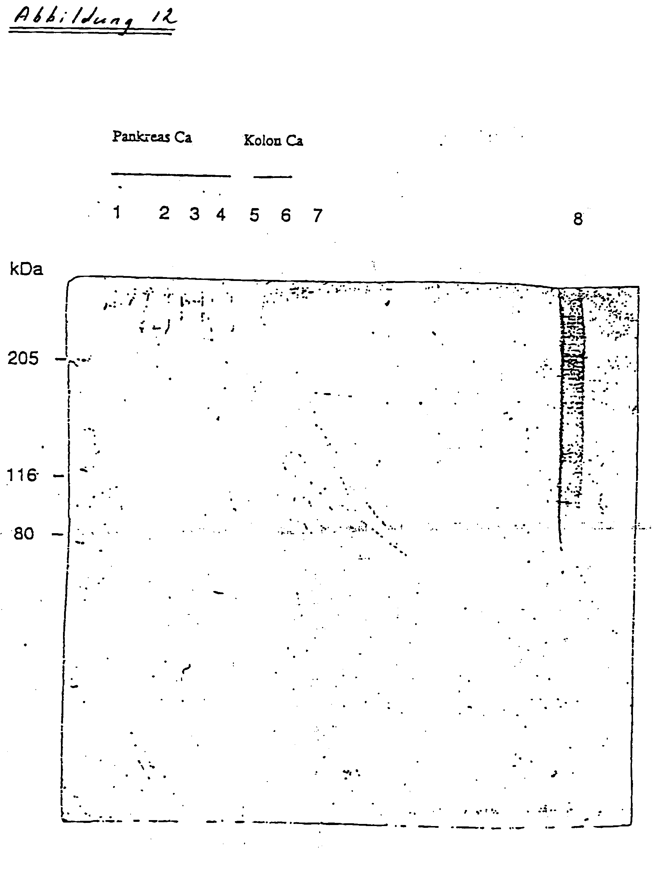

Immunoblots von Lysaten, die aus Bakterienkulturen (TB1) nach Transformation mit dem pMAL-c Vektor oder dem pMal-B1 Konstrukt isoliert wurden. Die Bakterien wurden vor und nach Induktion des Promotor durch IPTG geerntet und die Pellets in SDS-Gelladepuffer aufgenommen. Nach Auftrennung in einer 10% SDS-PAGE wurden die Proteine auf eine Nitrozellulosemembran transferiert und, wie für Abbildung 1 beschrieben, weiter verfahren. Spur 1: pMAL-B1 mit IPTG induziert; Spur 2: pMAL-c mit IPTG induziert; Spur 3: pMAL-B1 uninduziert; Spur 4: pMAL-c uninduziert;

### Abbildung 4:

Homologievergleich der Aminosäuresequenzen von zwei MBP-B1 Fusionsproteinen, die beide spezifisch durch den mAk B1 erkannt werden und deren Leserahmen um ein Nukleotid verschoben sind. Die identischen Aminosäurenmotive wurden einer Analyse zur Berechnung antigener Determinanten (Hopp und Woods, 1983) unterzogen, durch die ein Motiv QDREEE als potentielle antigene Determinante ermittelt werden konnte.

### Abbildung 5:

Northern-Blot Analyse mit verschiedenen humanen Tumorzellinien.
Die poly(A)⁺ angereinigten RNA Proben wurden in einem formaldehydhaltigen Agarosegel nach Molekulargewicht aufgetrennt und auf eine Nylonmembran transferiert. Als radioaktive Sonde wurde ein EcoRI/HindII Fragment des pB1 Klons verwendet (A). Die Normalisierung des Blots erfolgt mit einer β-Aktinsonde (B). Spur 1: HT-29; Spur 2: T84; Spur 3: CAMA; Spur 4: Mz-Sto-1; Spur 5: Capan-1;

### Abbildung 6:

Southern-Blot Analyse mit humaner, genomischer DNA.
Genomische DNA Proben (15 mg) von fünf verschiedenen Spendern (1-5) wurden mit EcoRI (a) oder MspI (b) vollständig verdaut und in einem 0,8% Agarosegel nach Molekulargewicht aufgetrennt. Als radioaktive Sonde wurde ein EcoRI/PstI 190 bp Fragment des pB1 Klons verwendet.

### Abbildung 7:

Nukleotidsequenz und abgeleitete Aminosäurensequenz des pB1.2 cDNA Klons. Die Sequenzen wurden nach dem gleichen Verfahren, wie für pB1 beschrieben, ermittelt.

### Abbildung 8:

Vollständige Nukleotidsequenz des genomischen Klons pB1-Cos im Vergleich mit den beiden cDNA Sequenzen pB1 und pB1.2. Die Exon/Intron Übergänge, Start- und Stopkodon sind durch Fettdruck hervorgehoben. Die nicht sequenzierten 225 bp im Exon sind unterstrichen. Das "Allignment" der Sequenzen wurde mittels der GeneWorks Software von IntelliGenetics durchgeführt.

### Abbildung 9:

Schematische Darstellung des pB1-Cos Exons und der beiden cDNA Sequenzen. Identische Bereich sind untereinander angeordnet. Die Darstellung ist nicht maßstabsgetreu.

### Abbildung 10:

Partialverdau der repetitiven Region des pB1-PCR Klons mit dem Restriktionsenzym Earl.
A: Agarosegel; B: Autoradiogramm des Southern-Blots mit dem EcoRI/HindII pB1 Fragment als radioaktive Sonde; Es wurden jeweils 2 mg Insert DNA unter folgenden Bedingungen verdaut: Spur 1: 2h 1U/mg; Spur 2: 5 min 0,5U/mg; Spur 3: 3 min 0,5 U/mg; Spur 4: 1 min 0,5U/mg; M: 123 bp Längenstandard;

### Abbildung 11:

Southern-Blot Analyse mit genomischer DNA von somatischen Mensch/Nager Zellhybriden.
Es wurden jeweils 10 mg der DNA Proben mit EcoRI vollständig verdaut und in einem 0,8 % Agarosegel aufgetrennt. Als radioaktive Sonde wurde das EcoRI/HindII pB1 Fragment verwendet. Die Proben wurden so aufgetragen, daß sich in Spur 1 DNA befindet, die das humane Chromosom 1 enthält, in Spur 2 DNA, die das humane Chromosom 2 usw.
Spur 25: humane Zellinie IMR91; Spur 26: Mauszellinie 3T6; Spur 27: Hamsterzellinie RJK88;

### Abbildung 12:

Western-Blot Analyse mit Seren von Patienten mit Pankreas- und Kolonkarzinom und einem gesunden Spender. Mz-Sto-1 Zellextrakt dient als Positivkontrolle. Von allen Seren wurden 10 ml aufgetragen. Als 1. Antikörper wurden 2 mg/ml mAk B1, als 2. Antikörper alkalische Phosphatase konjungierte anti-Maus Immunglobuline in einer 1:500 Verdünnung verwendet.

## Patentansprüche

1. cDNA kodierend für das Muzin MUC8 dadurch gekennzeichnet, daß sie im wesentlichen der Nucleotidsequenz pCos aus Abb. 8 von Nucleotid 3049 bis 4238 entspricht.

2. Rekombinante DNA dadurch gekennzeichnet, daß sie die cDNA gemäß Anspruch 1 enthält.

3. Muzin MUC8, das unter Verwendung der cDNA gemäß Anspruch 1 hergestellt wurde.

4. Muzin MUC8, das unter Verwendung der Aminosäuresequenz, die aus cDNA gemäß Anspruch 1 abgeleitet werden kann, hergestellt wurde.

5. cDNA gemäß Anspruch 1 dadurch gekennzeichnet, daß sie im wesentlichen der Nucleotidsequenz aus pCos aus Abb. 8 von Nucleotid 3049 bis 3325 entspricht.

6. cDNA gemäß Anspruch 1 dadurch gekennzeichnet, daß sie im wesentlichen der Nucleotidsequenz aus pCos aus Abb. 8 von Nucleotid 3326 bis 3666 entspricht.

7. cDNA gemäß Anspruch 1 dadurch gekennzeichnet, daß sie im wesentlichen der Nucleotidsequenz aus pCos aus Abb. 8 von Nucleotid 3667 bis 4238 entspricht.

8. DNA dadurch gekennzeichnet, daß sie im wesentlichen der Nucleotidsequenz pCos aus Abb. 8 entspricht.

9. DNA dadurch gekennzeichnet, daß sie im wesentlichen der Nucleotidsequenz pB 1.2 aus Abb. 8 entspricht.

10. DNA dadurch gekennzeichnet, daß sie im wesentlichen der Nucleotidsequenz pB1 aus Abb. 8 entspricht.

11. Diagnostisches Verfahren zum Nachweis und zur Bestimmung von humanen Antikörpern gegen das Muzin MUC8 als Tumormarker dadurch gekennzeichnet, daß das Muzin Peptide gemäß Anspruch 3 oder 4 oder daraus abgeleitete immunreaktive Peptide verwendet werden.

12. Verwendung des Muzins gemäß einem der Ansprüche 3 oder 4 oder von immunreaktiven Peptiden aus dem Muzin in einem Verfahren zum Nachweis von Antikörpern gegen das Muzin.

13. Verwendung gemäß Anspruch 11, worin der Nachweis mittels eines ELISA's erfolgt.

14. Verwendung des Muzins gemäß Anspruch 3 oder 4 oder daraus abgeleiteter immunreaktiver Peptide zur Erzeugung einer Antikörperbildung in einem Säugetier.

15. Verwendung gemäß Anspruch 14, wobei das Säugetier der Mensch ist.

16. Diagnostisches Verfahren zum Nachweis und zur Bestimmung von Muzin MUC8 als Tumormarker dadurch gekennzeichnet, daß die Sequenz pCos aus Abb. 8 oder daraus abgeleitete Teilsequenzen als Gensonden eingesetzt werden.

17. Verwendung der DNA gemäß einem der Ansprüche 1 und 2 und 5-10 zur rekombinanten Herstellung von Muzin MUC8 oder Teilsequenzen daraus.

18. Muzin MUC8 oder daraus abgeleitete Peptide, die aufgrund der Aminosäuresequenz, die aus der cDNA gemäß Anspruch 1 abgeleitet werden kann, synthetisch hergestellt werden kann.

19. Monoklonale oder polyklonale Antikörper gegen das Muzin MUC8, erhältlich nach Immunisierung mit MUC8 gemäß Anspruch 3 oder 4.

20. Diagnostikum zum Nachweis von MUC8, enthaltend einen oder mehrere Antikörper gegen MUC8 gemäß Anspruch 19.
